# EUROPÄISCHE PATENTSCHRIFT

(11) **EP 0 949 971 B1**
(45) Veröffentlichungstag und Bekanntmachung des Hinweises auf die Patenterteilung: **05.12.2001**
(21) Anmeldenummer: 97943853.8
(22) Anmeldetag: 09.09.1997
(51) Int. Cl.: B01J 35/06

(54) **VERWENDUNG VON MIKROPORÖSEN ANORGANISCHEN MEMBRANKATALYSATOREN**
USE OF MICROPOROUS ANORGANIC MEMBRANE CATALYSTS
UTILISATION DE CATALYSEURS MEMBRANAIRES INORGANIQUES MICROPOREUX

(30) Priorität: 13.09.1996 DE 19637365
(43) Veröffentlichungstag der Anmeldung: 20.10.1999
(73) Patentinhaber: Studiengesellschaft Kohle mbH, 45470 Mülheim an der Ruhr (DE)
(72) Erfinder: MAIER, Wilhelm, Friedrich, D-45470 Mülheim an der Ruhr (DE)
(74) Vertreter: von Kreisler, Alek, Dipl.-Chem.
(86) Internationale Anmeldenummer: EP9704918
(87) Internationale Veröffentlichungsnummer: WO9810865

(56) Entgegenhaltungen:
- EP-A- 0 228 885
- EP-A- 0 244 970
- US-A- 5 250 184
- US-A- 5 492 873

## Beschreibung

Folge- und Nebenreaktionen sind Hauptursache für reduzierte Ausbeuten und die Erzeugung von chemischem Abfall und Nebenprodukten in der chemischen Produktion. Es wurde nun gefunden, daß sich unerwünschte Folge- und Nebenreaktionen durch die Verwendung von mikroporösen Membrankatalysatoren unterdrücken und sogar ganz verhindern lassen.

Steigende Umweltauflagen und Kosten erhöhen zunehmend die Anforderungen an die chemische Produktion. Über 90 % der chemischen Produkte benötigen in einem oder mehreren Schritten heterogene Katalysatoren.

Eine neue Forschungsrichtung zur Verbesserung chemischer Prozesse sind die Membrankatalysatoren. Es handelt sich hier vorzugsweise um anorganische, katalytisch aktive Membranen, die gegenüber organischen Membranen den Vorteil höherer thermischer, chemischer und mechanischer Resistenz sowie grundsätzlich unbegrenzter Regenerierbarkeit und Sterilisierbarkeit haben, und sich auch bei höheren Temperaturen einsetzen lassen. Ihr Einsatz bewirkt die Verbesserung chemischer Produktionsprozesse durch die Kombination von Trenneigenschaften mit den katalytischen Eigenschaften. So läßt sich durch Membranen die Reaktionsführung so verändern, daß die flüssigen oder gasförmigen Reaktionspartner getrennt über die beiden Membranseiten strömen und dadurch nur im Inneren der Membran sich eine Reaktionszone ausbilden kann.. Die bislang bekannten Grundsätze und Merkmale solcher Membranreaktoren wurden in mehreren Übersichtsartikeln veröffentlicht (J.N. Armor, Appl. Catal 49 (1989), 1; H.P. Hsieh, Catal. Rev. Sci. Eng. 33 (1991) 1; M.P. Harold, P. Cini, B. Patenaude and K. Venkataraman, AIChE Symp. Ser 85 (268), 26 (1989)).

Die bevorzugte Permeation eines Reaktionspartner in porösen Membranen kann zur Selektivitätserhöhung genutzt werden (G. Saracco, V. Specchia, Catal. Rev. Sci. Eng. 36 (1994) 305). Meist wird versucht, durch selektive Abtrennung eines der Produkte oder selektive Zugabe eines der Ausgangsstoffe Reaktionsgleichgewichte und damit Selektivitäten und Ausbeuten zu verbessern

In Kontrast zu bisherigen Membrananwendungen wurde nun gefunden, daß überraschenderweise unerwünschte Folge- und Nebenreaktionen in verschiedenen chemischen Reaktionen durch die Verwendung von mikroporösen Membrankatalysatoren gezielt unterdrückt werden können, wenn die Porengröße der Membran nur geringfügig größer ist als die Reaktionspartner und wenn die Reaktion so durchführt wird, daß die Reaktionsmischung durch die Membran gepresst wird. Die katalytisch aktiven Zentren der Membran müssen im Inneren der Membran vorzugsweise auf der inneren Oberfläche der Poren lokalisiert sein. Signifikante Reaktivität auf der äußeren Membranoberfläche beeinträchtigt die Selektivität. Die hier beschriebene Erfindung unterscheidet sich von den genannten und anderen literaturbekannten Membrananwendungen vor allem dadurch, daß die Membran nicht zur permselektiven An- oder Abreicherungen von Produkten, Edukten oder Katalysatorgiften eingesetzt wird, sondern daß zwei oder mehr miteinander reagierende Edukte zusammen in die gleiche Richtung durch die katalytisch aktive Membran gepeßt werden. Durch die besondere Nanostruktur der Membran und die Reaktionsführung werden die Moleküle in den Poren vereinzelt und so Folgereaktionen verhindert. Diese Membrananwendung erlaubt damit erstmals, die Produktmoleküle schon während der Entstehung von den Ausgangsstoffen vollständig abzutrennen. Es handelt sich hier um eine Trennung in molekularen Dimensionen, die sich damit grundsätzlich von der Wirkung bekannter größerporiger Membranen unterscheidet.

Die Herstellung der Beschichtungslösungen der mikroporösen Katalysatormembranen erfolgt analog der Herstellung der Mischoxidkatalysatoren, wie in DE-A 195 45 042.6 und PCT/EP 96/00766 beschrieben. Ansonsten ist die Membranherstellung analog der in US PS 5,492,873 und US 5,250,184 beschriebenen Methoden. Aus diesen Anmeldungen kann man nicht entnehmen, daß bei geänderter Reaktionsführung und der Verwendung von Reaktanden, die nur geringfügig kleiner als die Poren der Membran sind, eine neue und effektive Methode zur Verhinderung von Folgereaktionen vorliegt.

Die Wirkungsweise der Unterdrückung von Folgereaktionen mit Memrankatalysatoren läßt sich wie folgt erläutern. Ein Reaktantmolekül A (z.B. Kohlenwasserstoff) reagiert mit einem Reaktionspartner B (z.B. Sauerstoff) am aktiven Zentrum des Katalysators unter Bildung des Zielproduktes Molekül C (z.B. ein Alkohol). Molekül C ist aber reaktiver als A (z.B. ein Alkohol ist bekannterweise reaktiver als ein Kohlenwasserstoff) und reagiert nun bevorzugt mit weiterem B unter rascher Bildung von Folgeprodukten D (z.B. Ketone, Carbonsäuren, Diole, usw. bis zu den Endprodukten Kohlendioxid und Wasser).

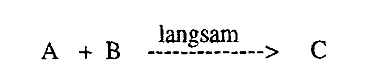

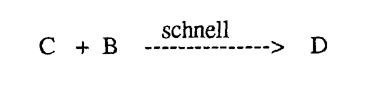

Dieses Problem ist bei vielen selektiven Oxidations-, Hydrierungs- und Halogenierungsreaktionen bekannt und wird in der Technik dadurch umgangen, daß die Reaktion im Unterschuß von B bei niedrigen Umsätzen von A und geringen Verweilzeiten gefahren wird. Verursacht wird das Problem durch die bei konventioneller Reaktionführung unvermeidliche Rückvermischung von Zielprodukt C.

Diese Rückvermischung läßt sich allerdings verhindern, wenn die Reaktion in den Poren einer Membran stattfindet und die Porengröße nicht größer als der doppelte kinetische Durchmesser dieser Moleküle ist. Da die meisten Moleküle für die heterogene Katalyse zwischen 0,3 und 1,5 nm groß sind, werden Porengrößen von mindestens 0,6 bis höchstens 3 nm je nach Molekülgröße benötigt. Essentiell ist dabei, daß eine sehr enge Porenverteilung der benötigten Porengröße vorliegt und daß sich genügend katalytisch aktive Zentren auf der inneren Oberfläche dieser Poren befinden.

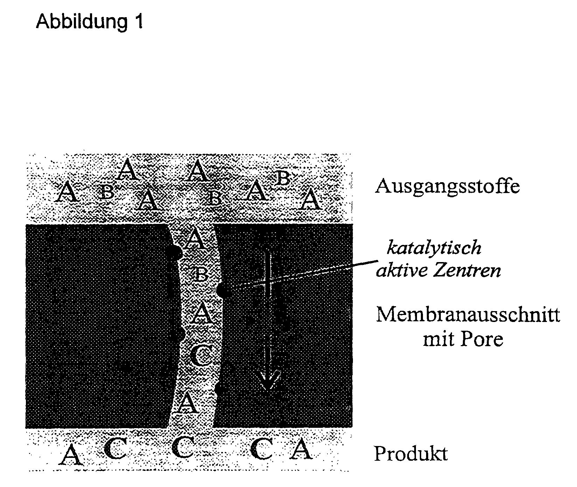

Abbildung 1 illustriert den Effekt solcher Poren. Oberhalb der Membran befindet sich das Gemisch/Lösung der beiden Reaktionspartner A und B. Diese strömen nun gemeinsam durch die Poren, wobei die Porengröße eine signifikante Veränderung der Vermischung während dieser Diffusion verhindert. Treffen während dieser Diffusion benachbarte A- und B-Moleküle auf ein aktives Zentrum, kann Umsetzung zu C stattfinden. Trifft C bei der weiteren Diffusion bis zum Austritt aus der Pore auf weitere katalytisch aktive Zentren, so kann keine weitere Umsetzung mehr stattfinden, da kein B mehr in nächster Nachbarschaft vorhanden ist und aufgrund der begrenzten Porendimension kein zusätzliches B mehr nachtransportiert werden kann. Jede kinetisch und thermodynamisch begünstigte Folgereaktion wird somit verhindert und eine hohe Selektivität zum Zielmolekül C erzielt. Dieser Effekt der verhinderten Rückvermischung ist deutlich nur bei korrekten Porengrößen der Membran erzielbar. Sind die Poren zu groß oder ist die Porenverteilung zu breit, wird die Produktselektivität durch unkontrollierte Diffusionseffekte beeinträchtigt.

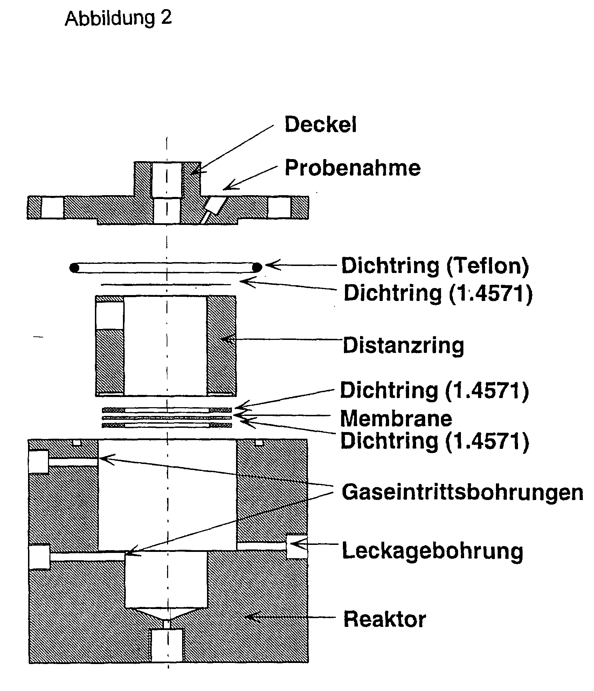

Der hier verwendete Reaktor ist in Abb. 2 aufgeführt. Die Reaktion läßt sich in allen Membranreaktoren, wie z.B. Röhrenreaktoren, Kapillarreaktoren und Kappillarbündelreaktor analog durchführen.

In den aufgeführten Beispielen wird die selektive Hydrierungen, selektive Oxidationen und selektive Alkylierung, durchgeführt an unterschiedlichsten Membranen, wie z.B. hydrophilen und hydrophoben Pt- und Pd-haltigen amorphen TiO2-Membranen, mikroporösen hydrophoben V-Si-Ti-Mischoxidmembranen und saueren Al-Si-Mischoxidmembranen erläutert.

Zur vollständigen Unterdrückung der Rückvermischung sind alle Membranen geeignet, sofern sie über eine monomodale Mikroporosität, aktive Zentren in den Poren und eine enge Porenverteilung mit Porendurchmessern nicht kleiner als 0,5 und nicht größer als 3 nm verfügen. Dies beinhaltet auch geeignete organische Membranen und defektfreie Zeolithmembranen. Als Reaktionen, deren Slektivität durch die verhinderte Rückvermischung verbessert werden kann, sind zu nennen Oxidationsreaktionen, Hydrierreaktionen, Chlorierungsreaktionen, Bromierungsreaktionen, Fluorierungsreaktionen, Additionsreaktionen, Cycloadditionsreaktionen, Oligomerisierungsreaktionen, Dimerisierungsreaktionen, aromatische und aliphatische Alkylierung- und Acylierungsreaktionen, Redoxreaktionen, pericyclische Reaktionen, Substitutionsreaktionen, Cyclisierungen, Hydrolysereaktionen, Eliminierungsreaktionen, Veresterungen und Veretherungen.

### Beispiel 1

### Herstellung einer hydrophoben Pt-haltigen Katalysatormembran:

### 1a) Herstellung der Beschichtungslösung:

In einem 20 ml Becherglas werden 0,105 g Na₂PtCl₆ in 10 ml Ethanol unter Rühren gelöst. In einem 100 ml Becherglas werden unter Argon 9,5 ml destilliertes Titan-(IV)-isopropoxid vorgelegt und anschließend 2,5 ml Methyltriethoxysilan (MTS) unter Rühren zugegeben. Nun werden 40 ml destilliertes Ethanol (im Abstand von 5 min jeweils 10 ml Ethanol) ins Becherglas gegeben. Nach 10 min Rühren werden sukzessive folgende Mengen Säure hinzugegeben: 0,1 ml 8 N HCl, nach 2 min Rühren 0,1 ml konz. HCl, nach weiteren 5 min Rühren 0,3 ml konz. HCl, nach weiteren 10 min Rühren 0,3 ml konz.HCl. Die Mischung wird unter Rühren mit dem Pt-Salz versetzt und nochmals mit 10 ml Ethanol verdünnt. Anschließend wird die Mischung für mehrere Stunden gerührt.

### 1b) Herstellung einer einfach beschichteten Katalysatormembran:

Die Katalysatormembran wird durch dip-coating als dünner Film auf eine kommerziell erhältliche Keramikmembran aufgebracht. In diesem Beispiel wurde eine kommerzielle asymmetrische Keramikmembran mit folgenden Merkmalen verwendet: Material Al₂O₃, Durchmesser der Scheiben: 47 mm, Dicke 2 mm, Trennschichtdicke 1,5 µm, mittlerer Porendurchmesser: 4,5 nm. Die Porengrößen und Porenverteilungen werden durch Aufnahme der Adsorptionsisotherme bei der Temperatur von flüssigem Argon oder flüssigem Stickstoff bestimmt. Die Keramikscheiben werden zunächst in einem Gemisch aus Isopropanol/Aceton 4 h am Rückfluß erhitzt und anschließend im Ofen bei 400 °C für 12 h getrocknet. Die so gesäuberten Scheiben werden wie folgt beschichtet.

Die Keramikscheibe wird dabei auf der großporigen Seite abgeklebt, an einem Faden befestigt und in obige Sol-Gel-Lösung 1a getaucht. In einer gesättigten Ethanolatmosphäre (geschlossene Apparatur) wird die Membran mit einer Zuggeschwindigkeit von 0,45 cm/min erschütterungsfrei aus der Lösung gezogen. Die Keramikmembran überzieht sich dabei mit einem dünnen Gelfilm. Nach dem vollständigen Herausziehen der Membran aus der Lösung wird der Faden von der Kupplung gelöst und mit Klebefilm am Deckel befestigt. Die Membran schwebt nun ca. 2 cm über dem Becherglas. Dieses wird durch kurzes Anheben des Zylinders entnommen. Nach der Entnahme des Becherglases wird 5 ml Ethanol in den Zylinder gespritzt und die Membran anschließend 5 Tage in der Alkoholatmosphäre hängen gelassen. Danach wird die Membran entnommen und schonend kalziniert. Um rißfreie, dünne Filme zu erhalten wird die Membran nach folgendem Temperaturprogramm getrocknet: 0,1 C /min Aufheizrate bis zu T = 65 C, halten von T = 65 C für 100 min, 0,1 C /min Aufheizrate bis zu T = 250 C, halten von T = 250 C für 300 min, abkühlen (10 C /min) auf Raumtemperatur. Die restliche Beschichtungslösung kann in der Tiefkühltruhe für weitere Beschichtungen aufgehoben oder zur Herstellung von pulverförmigen Katalysatoren weiter genutzt werden.

### 1c) Herstellung einer dreifach beschichteten Katalysatormembran:

Zur Erhöhung der Schichtdicke wurde die Beschichtung wie unter 1b) beschrieben dreimal ausgeführt.

### 1d) Herstellung eines pulverförmigen Pt-Vergleichskatalysators

Die restliche Beschichtungslösung von 1b oder 1c wurde 10h bei Raumtemperatur stehengelassen und dann wie unter 1b beschrieben getrocknet und kalziniert. Das dabei entstehende grobe Glaspulver wird mit der Pulvermühle auf die erforderliche Korngröße vermahlen und als Pulverkatalysator eingesetzt.

### Beispiel 2

### Selektive Hydrierung von 2-Hexin im Membranreaktor mit Membran 1b bei 50 °C

Die nach dem obigen Verfahren 1b hergestellte einmal beschichtete Membran wurde in einen Membranreaktor eingebaut und 12 h im Wasserstoffstrom (10 ml/min) bei einer Temperatur von 200 °C aktiviert. Anschließend wurde die Temperatur auf die Reaktionstemperatur (hier 50 °C) gesenkt. Der Reaktor wurde mit 10 ml n-Decan gefüllt und mit 200 µl 2-Hexin versetzt. Die Mischung wurde nun 2 min gerührt. Von oben wurde Wasserstoff aufgedrückt (Wasserstoffstrom von 30 ml/min bei Anschluß eines Blasenzählers oberhalb der Lösung). Der Reaktor wurde dicht verschlossen. Im Permeat waren bei einem Umsatz von 22 % nur 2- und 3-Hexene, kein 1-Hexene und kein n-Hexan nachweisbar (Empfindlichkeit < 0.1 %).

### Beispiel 3

### Selektive Hydrierung von 2-Hexin im Membranreaktor mit Membran 1b bei 110°C

Der Versuch wurde wie in 2. beschrieben durchgeführt. Bei einer Reaktionstemperatur von 110 °C ergab sich ein Umsatz von über 60 % im Permeat. Wiederum war im Permeat kein n-Hexan und kein 1-Hexen nachweisbar, als Produkte wurden nur isomere Hexene gebildet.

### Beispiel 4

### Selektive Hydrierung von 2-Hexin im Membranreaktor mit der dreifach beschichteten Membran 1c bei 110 °C

Der Versuch wurde wie in 2. beschrieben, aber mit Membran 1c durchgeführt. Im Permeat wurde ein 2-Hexin-Umsatz von über 60% erzielt. Wiederum war im Permeat kein n-Hexan und kein 1-Hexen nachweisbar, als Produkte wurden nur isomere Hexene gebildet.

### Beispiel 5

### Selektive Hydrierung von 1,3-Hexadien im Membranreaktor mit Membran 1b bei 110 °C

Die nach dem obigen Verfahren 1b hergestellte einmal beschichtete Membran wurde in einen Membranreaktor eingebaut und 12 h im Wasserstoffstrom (10 ml/min) bei einer Temperatur von 200 °C aktiviert. Anschließend wurde die Temperatur auf 90 °C gesenkt. Der Reaktor wurde mit 10 ml n-Decan gefüllt und mit 200 µl 1,3 Hexadien versetzt. Die Mischung wurde nun 2 min gerührt. Von oben wurde Wasserstoff aufgedrückt (Wasserstoffstrom von 30 ml/min bei Anschluß eines Blasenzählers oberhalb der Lösung). Der Reaktor wurde dicht verschlossen. Im Permeat waren bei einem Umsatz von > 80 % nur t-2-Hexen und c-3-Hexen (15:85), kein 1-Hexene und kein n-Hexan nachweisbar (Empfindlichkeit <0.1%).

### Beispiel 6

### Hydrierung von 2-Hexin im Satzreaktor

Der Katalysator 1d wurde vor beginn der Reaktion im Wasserstoffstrom bei 200°C aktiviert. In 50 ml n-Decan wurden 0,5 ml 2-Hexin und 100 mg Katalysator 1d (Korngröße < 100 µm) im Satzreaktor (150 ml Kolben) bei 90 °C in einer H₂-Atmosphäre mit 2000 U/min gerührt. Der Reaktionsverlauf wurde mit dem Gaschromatographen verfolgt. Schon bei niedrigen Umsätzen von 4 % waren über 70 % des Produktes n-Hexan, 26 % 2-Hexene und 4 % t-3-Hexen.

### Beispiel 7

### Hydrierung von 1,3-Hexadien im Satzreaktor

Der Katalysator 1d wurde vor Beginn der Reaktion im Wasserstoffstrom bei 200 °C aktiviert. In 50 ml n-Decan wurden 0,5 ml 2-Hexin und 100 mg Katalysator 1d (Korngröße < 100 µm) im Satzreaktor (150 ml Kolben) bei 90 °C in einer H₂-Atmosphäre mit 2000 U/min gerührt. Der Reaktionsverlauf wurde mit dem Gaschromatographen verfolgt. Auch hier war n-Hexan schon bei Umsätzen von < 10 % das dominierende Produkt.

### Beispiel 8

### Herstellung einer Pd-haltigen TiO₂-Membran

Die Herstellung der Beschichtungslösung erfolgte analog der Beschreibung 1a, nur wurden anstatt des Na₂PtCl₆ Pt-salzes hier 0,035 g Pd-(II)-acetylacetonat (21,5 mmol) eingesetzt. Die Herstellung der Membran wurde analog der unter 1b beschriebenen Herstellung der Pt-haltigen Membran durchgeführt.

### Beispiel 9

### Hydrierung von 2-Hexin an der Pd-haltigen Membran 8

Die nach dem obigen Verfahren 8 hergestellte einmal beschichtete Membran wurde in einen Membranreaktor eingebaut und 12 h im Wasserstoffstrom (10 ml/min) bei einer Temperatur von 200 °C aktiviert. Anschließend wurde die Temperatur auf 110 °C gesenkt. Der Reaktor wurde mit 10 ml n-Decan gefüllt und mit 100 µl 2-Hexin versetzt. Die Mischung wurde nun 2 min gerührt. Von oben wurde Wasserstoff aufgedrückt (Wasserstoffstrom von 30 ml/min bei Anschluß eines Blasenzählers oberhalb der Lösung). Der Reaktor wurde dicht verschlossen. Im Permeat befanden sich bei einem Umsatz von 46 % nur Hexene. Es war kein n-Hexan nachweisbar (Empfindlichkeit < 0.1 %).

### Beispiel 10

### Herstellung einer Katalysatormembran für die saure Katalyse

### a) Vorbeschichtung der Membran

Als Trägermembran wurde eine kommerzielle keramische Membran mit folgenden Merkmalen verwendet: Durchmesser 5 cm, Dicke 2 mm, Porendurchmesser <1 µm. Diese Membran wurde analog 1b gereinigt und 2 mal analog 1b mit folgender Lösung beschichtet: 50 ml Ethanol wurden mit 40 ml TEOS versetzt und unter Rühren wurden innerhalb von 10 min eine Lösung aus 4 mg Ammoniumfluorid in 8 ml destilliertem Wasser zugetropft. Diese Lösung wurde bei Raumtemperatur für weitere 2 h gerührt und dann für die doppelte Beschichtung der Trägermembran analog 1b eingesetzt. Nach dem Brennen der Membran wurde folgende Sol-Gel-Beschichtungslösung hergestellt.

### b) Membranherstellung

20 ml TEOS (0,0783 mol) werden in einem 150 ml Polypropylenbecher mit 25 ml Ethanol gelöst und tropfenweise mit einer Lösung aus 4ml H₂O und 0,87g Al(NO₃)₃ (2,3mmol) versetzt.
Die Lösung wird 5 min gerührt und mit 500µl BF₃/Acetatkomplex angesäuert. Mit der so gewonnenen Sol-Gel-Lösung wird die vorbeschichtete Membran nach dem Dipcoatingverfahren 1b beschichtet. Die Membran wird anschließend 5 Tage in einer ethanolischen Atmosphäre getrocknet und schonend kalziniert. Um Rückvermischungen zu minimieren wird die aktive aluminiumhaltige Schicht noch einmal mit einer inaktiven SiO₂ Schicht analog 10a überzogen.

### Beispiel 11

### Ethylierung von Biphenyl mit Ethanol an der Katalystormembran 10

Die nach dem obigen Verfahren 10 hergestellte Membran wurde in einen Membranreaktor eingebaut und in einem Argonstrom (290 ml/min) mit 1°C/min auf die Reaktionstemperatur von 250 °C erwärmt. Biphenyl wurde in einem separaten Feststoffverdampfer auf 140 °C erhitzt und mit einem Ethylengasstrom von 10 ml/min kontinuierlich verdampft und durch die Membran geleitet. Das Permeat wurde mit Trockeneis gekühlt und die erhaltenen Feststoffe wurden mit GC analysiert. Als Produkt wurden nur monoethylierte Biphenyle erhalten.
Die Isomere verteilen sich zu 41% auf 2-Ethylbiphenyl, 32% auf 3-Ethylbiphenyl und 27% auf 4-Ethylbiphenyl.

### Beispiel 12

### Herstellung einer hydrophoben vanadiumhaltigen Katalysatormembran

Die asymetrische Trägermembran wird analog Beispiel 1 beschichtet. Zur Herstellung der Beschichtungslösung werden 1,9 g Vanadium-(II)-acetylacetonat, 25,3 mL TEOS, 9,7 mL MTES, 29,0 mL EtOH und 7,21 mL 8 N HCl in einem PP-Becher 1 h gerührt und dann wie unter 1b beschrieben hergestellt.

### Beispiel 13

### Selektive Cyclohexanoxidation an der Katalysatormembran 12 mit TBHP

Die in den Membranreaktor eingebaute Membran 12 wurde im Ar-Strom bei 200 °C über Nacht ausgeheizt und dann auf 90 °C abgekühlt. Auf die Membran wurden 3,04 mL Cyclohexan und 6,95 mL TBHP (3 M Lösung in Isooctan) gegeben (molares Verhältnis von TBHP zu Cyclohexan = 2 / 1). Im Permat waren bei einem Umsatz von 70% nur Cyclohexanol und Cyclohexanon im Verhältnis 1:1 vorhanden. Durch Erhöhung des Druchflusses wurde eine Reduktion des Umsatzes und eine Erhöhung des Verhältnisses Cyclohexanol/Cyclohexanon auf bis zu 1,7 derzielt.

### Beispiel 14

### Epoxidation von 1-Octen mit TBHP im Membranreaktor mit Membrane 12

Der Membranreaktor wurde mit eingesetzter vanadiumhaltiger Membran 12 auf 200°C mit 1°C/min aufgeheizt. Dabei wurde der Reaktor mit 90°C heißem Argon gespült. Die Reaktortemperatur wurde für 1h gehalten. Anschließend ließ man mit 0.1°C/min im Argonstrom auf Raumtemperatur abkühlen. 1-Octen (47.4 mmol, 5.32 g, 7.44 ml) und t-butylhydroperoxid (3M, wasserfrei in Isooctan, 9.0 mmol, 2.27 g, 3.00 ml) wurden nacheinander in den Reaktor gegeben. Der Reaktor wurde verschlossen und unter Rühren (300 U/min) auf 80°C aufgeheizt. Die Entnahme von Proben erfolgte durch das Probenentnahmeventil unterhalb der Membran.
Das Permeat zeigte bei einem Umsatz von 11 % eine Produktselektivität von < 99 % zu 1-Epoxyoctan, dem einzigen Produkt.

### Beispiel 15

### Herstellung einer hydrophilen mikroporösen Pt-haltigen Katalysatormembran

a) In einem 20 ml Becherglas werden 0,105 g Na₂PtCl₆ in 10 ml Ethanol unter Rühren gelöst.
   In einem 100 ml Becherglas werden unter Argon 12 ml destilliertes Titan-(IV)-isopropoxid unter Rühren vorgelegt. Nun werden 40 ml destilliertes Ethanol (im Abstand von 5 min jeweils 10 ml Ethanol) zugegeben. Nach 10 min Rühren werden sukzessive folgende Mengen Säure hinzugegeben: 0,1 ml 8 N HCl, nach 2 min Rühren 0,1 ml konz. HCl, nach weiteren 5 min Rühren 0,3 ml konz. HCl, nach weiteren 10 min Rühren 0,3 ml konz.HCl. Die Mischung wird unter Rühren mit dem Pt-Salz versetzt und nochmals mit 10 ml Ethanol verdünnt. Anschließend wird die Mischung für mehrere Stunden gerührt.
b) Als Trägermembran wurde eine vorbeschichtete Membran wie unter 10a beschreiben eingesetzt. Die Membranherstellung erfolgte mit der Beschichtungslösung 16a, aber anonsten wie unter 1b beschreiben.

### Beispiel 16

### Selektive Hydrierung von 2-Hexin im Membranreaktor mit Membran 16 bei 60 °C

2-Hexin wurde an der Membran wie unter 2 beschreiben bei einer Reaktionstemperatur von 60 °C umgesetzt. Im Permeat war bei einem Umsatz von 10 % nur cis-2-Hexen als einziges Produkt nachweisbar.

## Patentansprüche

1. Verfahren zur Durchführung von katalysierten chemischen Reaktionen unter Verwendung von amorphen mikroporösen Membranen, **dadurch gekennzeichnet, daß** als amorphe mikroporöse Membranen solche mit Porengrößen von 0,5 bis 3 nm mit einer Schichtdicke unter 10 µm und einer Halbwertsbreite der Porenverteilung von < 0,3 nm, die in ihren Poren wenigstens eine katalytisch aktive Komponente für heterogen katalysierte Reaktionen enthalten, verwendet werden, wobei zwei oder mehr miteinander reagierende Ausgangsprodukte gleichzeitig in gleicher Richtung durch die katalytischen Membranen gepreßt werden.

2. Verfahren nach Anspruch 1, **dadurch gekennzeichnet, daß** die Durchmesser der Poren der verwendeten Membranen nicht größer als der doppelte Durchmesser der Moleküle der Ausgangsprodukte sind und die Dicke der amorphen Schicht von 0,1 bis unter 10 µm beträgt, wobei zwei oder mehr Reaktanden unter damit verbundener Vermeidung einer Rückmischung des Reaktionsproduktes durch die Membranen geführt werden.

3. Verfahren nach Anspruch 2, **dadurch gekennzeichnet, daß** die Porengröße 0,6 bis 3 nm beträgt.

4. Verfahren nach Ansprüchen 2 oder 3, **dadurch gekennzeichnet, daß** die Dicke der amorphen Schicht < 2 µm beträgt.

## Claims

1. A process for performing catalyzed chemical reactions using amorphous microporous membranes, **characterized in that**, as said amorphous microporous membranes, those are used which have pore sizes of from 0.5 to 3 nm with a layer thickness of below 10 µm and a half-width of the pore distribution of < 0.3 nm and which contain in their pores at least one catalytically active component for heterogeneously catalyzed reactions, wherein two or more mutually reacting starting materials are simultaneously pressed in the same direction through the catalytic membranes.

2. The process according to claim 1, **characterized in that** the diameters of the pores of the membranes employed are not larger than twice the diameter of the molecules of said starting materials, and the thickness of the amorphous layer is from 0.1 to below 10 µm, wherein two or more reactants are passed through the membrane while avoiding the back mixing of the reaction product.

3. The process according to claim 2, **characterized in that** said pore size is from 0.6 to 3 nm.

4. The process according to claims 2 or 3, **characterized in that** the thickness of the amorphous layer is < 2 µm.

## Revendications

1. Procédé pour mettre en oeuvre des réactions chimiques catalysées, par utilisation de membranes microporeuses amorphes, **caractérisé en ce qu'**on utilise en tant que membranes microporeuses amorphes des membranes ayant un diamètre des pores de 0,5 à 3 nm pour une épaisseur de couche inférieure à 10 µm et une largeur à mi-hauteur de la répartition des pores < 0,3 nm, membranes qui dans leurs pores contiennent au moins un composant catalytiquement actif pour des réactions à catalyse hétérogène, dans lequel au moins deux produits de départ, réagissant mutuellement, ou plus, sont comprimés simultanément et dans la même direction à travers les membranes catalytiques.

2. Procédé selon la revendication 1, **caractérisé en ce que** le diamètre des pores des membranes utilisées n'est pas supérieur au double du diamètre des molécules des produits de départ, et que l'épaisseur de la couche amorphe est de 0,1 à moins de 10 µm, deux réactifs ou plus étant envoyés à travers les membranes, tout en évitant un mélange inverse, qui y est associé, du produit de la réaction.

3. Procédé selon la revendication 2, **caractérisé en ce que** le diamètre des pores est de 0,6 à 3 nm.

4. Procédé selon la revendication 2 ou 3, **caractérisé en ce que** l'épaisseur de la couche amorphe est < 2 µm.
